# EUROPEAN PATENT APPLICATION

(11) **EP 4 717 691 A1**
(43) Date of publication of application: **01.04.2026**
(21) Application number: 23937866.4
(22) Date of filing: 22.05.2023
(51) Int. Cl.: C07D 241/38, C07D 403/14, A61K 31/416, A61K 31/4162, A61P 25/00

(54) **USE OF BMPR1B INHIBITOR IN INDUCED DEVELOPMENT, AND INDUCTION CULTURE MEDIUM**

(71) Applicant: Iregene Therapeutics Co., Ltd, Chengdu, Sichuan 610200 (CN)
(72) Inventor: WEI, Jun, Suzhou, Jiangsu 215021 (CN); CHEN, Xiping, Suzhou, Jiangsu 215021 (CN); ZHU, Yasha, Suzhou, Jiangsu 215021 (CN); YAN, Ruorong, Suzhou, Jiangsu 215021 (CN); CAI, Meng, Suzhou, Jiangsu 215021 (CN)
(74) Representative: Metida
(86) International application number: PCT/CN2023/095448
(87) International publication number: WO 2024/239187

(57) **Abstract**

The present application relates to the technical field of biological medicines, and in particular to a use of a BMPR1B inhibitor in induction development, an induction culture medium, and an induction method. The induction culture medium comprises a BMPR1B inhibitor having a structure represented by formula I, or a pharmaceutically acceptable salt, ester, amide, solvate, active metabolite, polymorph, isotope marker, isomer or prodrug of the structure represented by formula I.

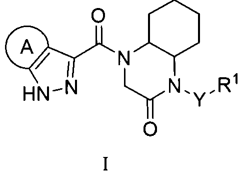

## Description

### Background of the Invention

### Field of the Invention

The present application relates to the field of biomedicine, and in particular to the use of a BMPR1B inhibitor in induction of development, as well as to an induction medium.

### Description of Related Art

Transforming growth factor-β (TGF-β) belongs to the TGF-β superfamily and plays a critical role in regulating cell growth and differentiation. The cell membrane receptors of TGF-β are mainly classified into two types: type I receptors and type II receptors. In vertebrates, seven type I receptors, known as activin-receptor-like kinases (ALKs), have been identified: ALK1 (ACVRL1), ALK2 (ACVR1), ALK3 (BMPR1A), ALK4 (ACVR1B), ALK5 (TGFBR1), ALK6 (BMPR1B), and ALK7 (ACVR1C); and five type II receptors: TGFβRII, ACTRII, ACTRII B, BMPRII, and AMHRII.

Among them, ALK6, which belongs to the type I receptors, corresponds to BMPR1B (bone morphogenetic protein receptor, type IB), an important transmembrane receptor protein. BMPR1B plays a key role in regulating osteogenic differentiation, cell proliferation, and ovarian follicle development. Therefore, the artificial modulation of BMPR1B to control downstream gene expression and physiological processes has become a significant research focus in the field of biomedicine.

### Summary of the Invention

Based on this, it is necessary to provide an induction medium, an induction method, and the use of a BMPR1B inhibitor.

In a first aspect, the present application provides an induction medium comprising a BMPR1B inhibitor, the BMPR 1B inhibitor has a structure represented by Formula I, or a pharmaceutically acceptable salt, ester, amide, solvate, active metabolite, polymorph, isotopically labeled compound, isomer, or prodrug thereof; 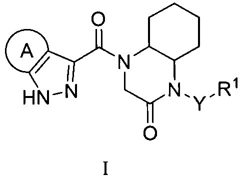 wherein the ring A is selected from any one of the following structures: 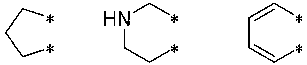
Y is -(CH₂)ₙ-, where n is 0, 1, 2, or 3;
R¹ is selected from -H, -D, unsubstituted or R²-substituted C₁-C₆ alkyl, C₁-C₆ alkoxy, C₆-C₁₀ aryl, or C₃-C₁₀ heteroaryl;
R² is selected from -F, -Cl, -Br, -I, -OH, -COOH, C₁-C₆ alkyl, or C₁-C₆ alkoxy;
"*" represents a point of attachment.

In a second aspect, the present application provides a method for inducing the differentiation of pluripotent stem cells into neural lineage cells, comprising the following step: adherently culturing pluripotent stem cells in the aforementioned induction medium to obtain neural lineage cells.

In a third aspect, the present application provides the use of the aforementioned BMPR1B inhibitor in inducing the differentiation of pluripotent stem cells into neural lineage cells.

### Brief Description of the Figures

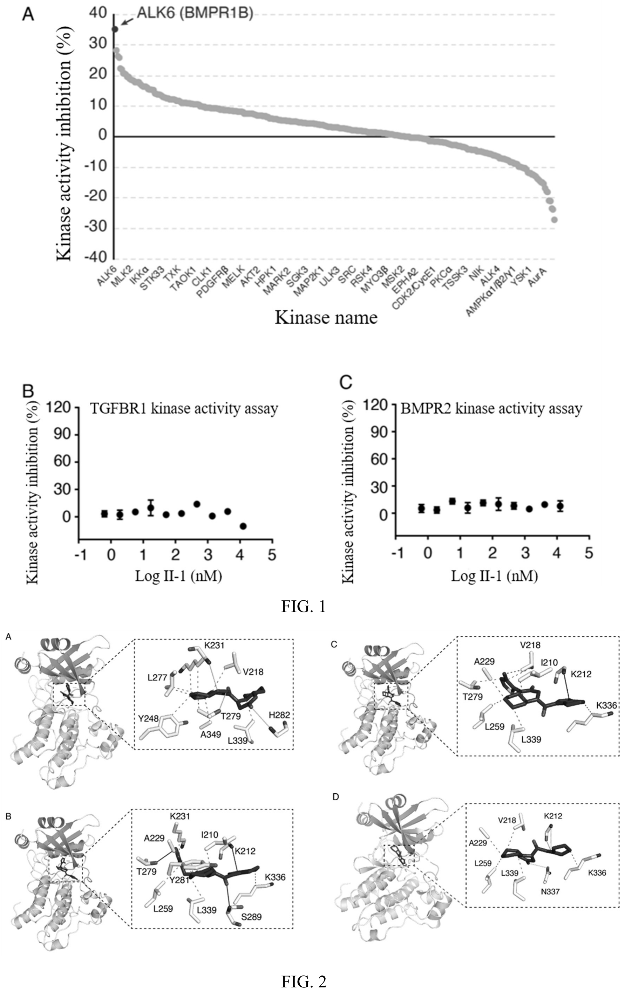

Figure 1 shows the specificity of pyrazolecarbonyl piperazinone compounds binding to their target based on kinase activity screening analysis. Figure 1A presents the effect of compound II-1 on the enzymatic activities of 330 kinases. Figures 1B and 1C show the kinase activity assay results of compound II-1 against TGFBR1 and BMPR2 at different concentrations.
Figure 2 presents the interactions between pyrazolecarbonyl piperazinone compounds and BMPR1B in the complex as visualized by PyMol software. In Figure 2, the compounds are: II-13 in 2A, II-11 in 2B, II-2 in 2C, and II-1 in 2D.

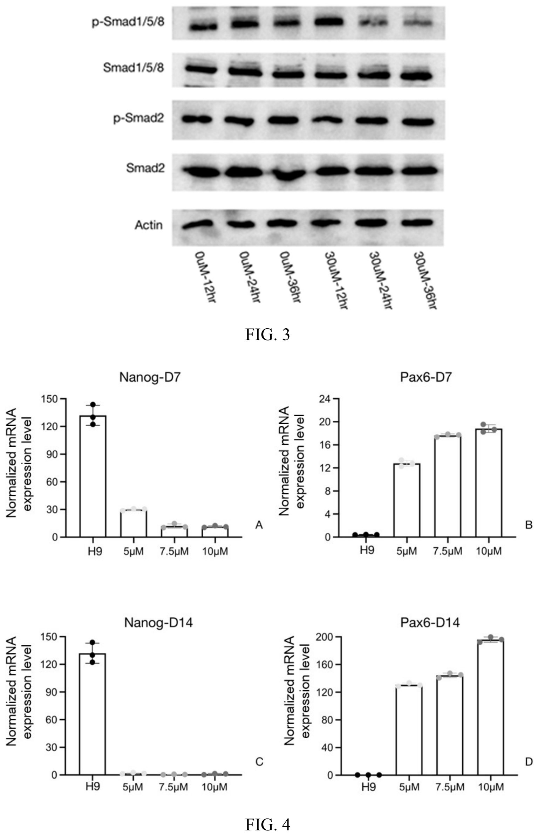

Figure 3 illustrates the details of the Western blot experiment in Example 3, where "12hr" represents 12 hours, "24hr" represents 24 hours, and "36hr" represents 36 hours.
Figure 4 shows the changes in mRNA expression levels of marker genes in H9 cells treated with different concentrations of compound II-1. Figure 4A shows NANOG mRNA levels after 7 days of treatment; Figure 4B shows PAX6 mRNA levels after 7 days; Figure 4C shows NANOG mRNA levels after 14 days; and Figure 4D shows PAX6 mRNA levels after 14 days.

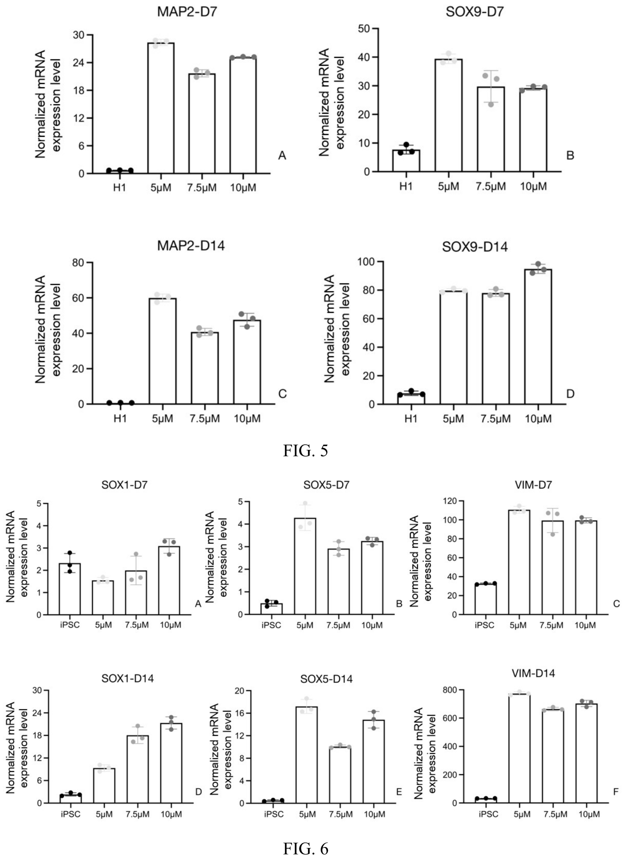

Figure 5 shows the changes in mRNA expression levels of marker genes in H1 cells treated with different concentrations of compound II-13. Figure 5A shows MAP2 mRNA levels after 7 days of treatment; Figure 5B shows SOX9 mRNA levels after 7 days; Figure 5C shows MAP2 mRNA levels after 14 days; and Figure 5D shows SOX9 mRNA levels after 14 days.
Figure 6 shows the changes in mRNA expression levels of marker genes in induced pluripotent stem cells (iPSCs) treated with different concentrations of compound II-13. Figure 6A shows SOX1 mRNA levels after 7 days; Figure 6B shows SOX5 mRNA levels after 7 days; Figure 6C shows VIMENTIN (VIM) mRNA levels after 7 days; Figure 6D shows SOX1 mRNA levels after 14 days; Figure 6E shows SOX5 mRNA levels after 14 days; and Figure 6F shows VIMENTIN (VIM) mRNA levels after 14 days.

### Detailed Description of the Invention

For the purpose of facilitating the understanding of the present application, the following provides a more detailed description of the present application with reference to the accompanying drawings. The drawings illustrate preferred embodiments of the present application. However, the present application may be implemented in many different forms and is not limited to the embodiments described herein. Rather, the purpose of providing these embodiments is to enable a more thorough and comprehensive understanding of the disclosure of the present application.

Unless otherwise defined, all technical and scientific terms used herein have the same meanings as those commonly understood by persons skilled in the art to which the present application pertains. The terminology used in the specification of the present application is solely for the purpose of describing particular embodiments and is not intended to limit the present application. The term "and/or" as used herein includes any and all combinations of one or more of the listed items.

In the present application, an open-ended description of technical features includes both a closed-ended technical solution composed of the listed features and an open-ended technical solution comprising the listed features.

In the present application, numerical ranges are considered continuous and include the minimum and maximum values of the range, as well as any value therebetween, unless otherwise specified. Furthermore, when the range refers to integers, it includes every integer between the minimum and maximum values. In addition, when multiple ranges are described for a feature or property, the ranges may be combined. In other words, unless otherwise indicated, all ranges disclosed herein are understood to include any and all sub-ranges therein.

Percentages disclosed in the present application, unless otherwise specified, refer to weight percent for solid-liquid and solid-solid mixtures, and volume percent for liquid-liquid mixtures.

Percentage concentrations disclosed herein, unless otherwise specified, refer to the final concentration, which is the proportion of the added component in the system after addition.

Temperature parameters disclosed herein, unless otherwise specified, allow for both constant-temperature treatment and treatment within a certain temperature range. Constant-temperature treatment permits fluctuations within the precision range of the instrument.

### Terminology Definitions

The term "alkyl" refers to a monovalent residue derived from a saturated hydrocarbon by removal of a hydrogen atom, wherein the carbon atom may be primary, secondary, tertiary, quaternary, or a combination thereof. Phrases containing this term, such as "C₁-C₆ alkyl," refer to an alkyl group containing 1 to 6 carbon atoms, and each occurrence may independently be a C₁, C₂, C₃, C₄, C₅, or C₆ alkyl. Suitable examples include, but are not limited to: methyl (Me, -CH₃), ethyl (Et, -CH₂CH₃), 1-propyl (n-Pr, -CH₂CH₂CH₃), 2-propyl (i-Pr, -CH(CH₃)₂), 1-butyl (n-Bu, -CH₂CH₂CH₂CH₃), 2-methyl-1-propyl (i-Bu, -CH₂CH(CH₃)₂), 2-butyl (s-Bu, -CH(CH₃)CH₂CH₃), 2-methyl-2-propyl (t-Bu, -C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), and 3,3-dimethyl-2-butyl (-CH(CH₃)C(CH₃)₃.

The term "alkoxy" refers to a group of the structure -O-alkyl, i.e., an alkyl group as defined above linked via an oxygen atom to an adjacent moiety. Phrases containing this term, such as "C₁-C₆ alkoxy," refer to an alkyl portion containing 1-6 carbon atoms, and each occurrence may independently be C₁, C₄, C₅, or C₆ alkoxy. Suitable examples include, but are not limited to: methoxy (-O-CH₃ or -OMe), ethoxy (-O-CH₂CH₃ or - OEt), and tert-butoxy (-O-C(CH₃)₃ or -OtBu).

The term "aryl" refers to an aromatic hydrocarbon group derived from an aromatic ring compound by removal of a hydrogen atom and may be monocyclic, fused, or polycyclic, with at least one aromatic ring in the system for polycyclic structures. For example, "C₆-C₁₀ aryl" refers to an aryl group containing 6-10 carbon atoms, each occurrence independently being C₆, C₇, C₈, C₉, or C₁₀ aryl. Suitable examples include, but are not limited to: phenyl, biphenyl, naphthyl, and their derivatives.

The term "heteroaryl" refers to an aryl or cyclopentadienyl group in which at least one carbon atom is replaced by a non-carbon atom such as N, O, or S. For example, "C₃-C₁₀ heteroaryl" refers to a heteroaryl group containing 3-10 carbon atoms, each occurrence independently being C₃, C₄, C₅, C₆, C₇, or C₈ heteroaryl. Suitable examples include, but are not limited to: furan, benzofuran, thiophene, benzothiophene, pyrrole, pyrazole, triazole, imidazole, oxazole, isoxazole, thiazole, tetrazole, indole, carbazole, pyrrolo[1,2-a]imidazole, pyrrolo[1,2-a]pyrrole, thieno[2,3-b]pyrrole, thieno[3,2-b]thiophene, furano[2,3-b]pyrrole, furano[2,3-b]furan, thieno[2,3-b]furan, benzoisoxazole, benzoisothiazole, benzimidazole, pyridine, pyrazine, diazine, pyrimidine, triazine, quinoline, isoquinoline, naphthyridine, quinoxaline, phthalazine, quinazoline, and quinazolinone.

The term "prodrug" refers to any compound that, upon administration to a biological system, undergoes spontaneous chemical reaction, enzymatic transformation, photolysis, and/or metabolic chemical reaction to produce the active drug, i.e., the active ingredient. Prodrugs are thus covalently modified analogs or latent forms of therapeutically active compounds. Suitable examples include, but are not limited to: carboxylic esters, carbonates, phosphate esters, nitrate esters, sulfate esters, sulfonates, sulfamates, amino compounds, carbamates, azo compounds, phosphoramides, glycosides, ethers, acetal derivatives, and the like.

The term "pharmaceutically acceptable" refers to ligands, materials, compositions, and/or dosage forms that are suitable for administration to a patient within the bounds of reasonable medical judgment and having a reasonable benefit/risk ratio.

The term "pharmaceutically acceptable carrier" refers to pharmaceutically acceptable materials, compositions, or vehicles, such as liquid or solid fillers, diluents, excipients, solvents, or encapsulating materials. As used herein, the term "pharmaceutically acceptable carrier" includes buffers, sterile water for injection, solvents, dispersing media, coatings, antibacterial and antifungal agents, isotonic agents, absorption retardants, and the like, which are compatible with the administration of the drug. Each carrier must be "pharmaceutically acceptable" in the sense of being compatible with other ingredients of the formulation and non-toxic to the patient. Suitable examples include, but are not limited to: (1) sugars such as lactose, glucose, and sucrose; (2) starches such as corn starch, potato starch, and substituted or unsubstituted β-cyclodextrins; (3) cellulose and derivatives such as sodium carboxymethylcellulose, ethylcellulose, and cellulose acetate; (4) powdered tragacanth; (5) malt; (6) gelatin; (7) talc; (8) excipients such as cocoa butter and suppository waxes; (9) oils such as peanut oil, cottonseed oil, safflower oil, sesame oil, olive oil, corn oil, and soybean oil; (10) glycols such as propylene glycol; (11) polyols such as glycerol, sorbitol, mannitol, and polyethylene glycol; (12) esters such as ethyl oleate and ethyl laurate; (13) agar; (14) buffers such as magnesium hydroxide and aluminum hydroxide; (15) alginates; (16) pyrogen-free water; (17) isotonic saline; (18) Ringer's solution; (19) ethanol; (20) phosphate-buffered saline; and (21) other non-toxic compatible substances used in pharmaceutical formulations.

The term "pharmaceutically acceptable salt" refers to any salt of the compounds disclosed herein with acids or bases suitable for use as a medicament. Pharmaceutically acceptable salts include inorganic and organic salts. One type of salt is a salt of the disclosed compound with an acid. Suitable acids include, but are not limited to: inorganic acids such as hydrochloric acid, hydrobromic acid, hydrofluoric acid, sulfuric acid, nitric acid, and phosphoric acid; organic acids such as formic acid, acetic acid, trifluoroacetic acid, propionic acid, oxalic acid, malonic acid, succinic acid, fumaric acid, maleic acid, lactic acid, malic acid, tartaric acid, citric acid, benzoic acid, methanesulfonic acid, ethanesulfonic acid, p-toluenesulfonic acid, benzenesulfonic acid, naphthalenesulfonic acid; and amino acids such as proline, phenylalanine, aspartic acid, glutamic acid. Another type of salt is a salt of the disclosed compound with a base. Suitable bases include, but are not limited to: alkali metal salts (e.g., sodium or potassium), alkaline earth metal salts (e.g., magnesium or calcium), ammonium salts (e.g., lower alkanol ammonium salts and other pharmaceutically acceptable amine salts) such as methylamine, ethylamine, propylamine, dimethylamine, trimethylamine, diethylamine, triethylamine, tert-butylamine, ethylenediamine, hydroxyethylamine, dihydroxyethylamine, tri(hydroxyethyl)amine, and amine salts derived from morpholine, piperazine, or lysine.

The term "pharmaceutically acceptable ester or amide" refers to any ester or amide of the disclosed compounds suitable for use as a medicament. Pharmaceutically acceptable esters include organic and inorganic esters.

In cases where the compound of Formula I contains a carboxyl group, pharmaceutically acceptable esters or amides can be prepared by conventional methods. For example, the compound of Formula I or its active derivative (e.g., an acyl chloride, mixed anhydride, or the like) can be reacted with a suitable alcohol (e.g., a C₁-C₆ alcohol) or its active derivative (e.g., (5-methyl-2-oxo-1,3-dioxolen-4-yl)methyl, neopentyl, benzofuranone, [(isopropoxycarbonyl)oxy]methyl, or the like), or with ammonia or a suitable amine (e.g., a mono-C₁-C₆ alkylamine or a di-C₁-C₆ alkylamine). Similarly, the ester of the compound of Formula I can be reacted with a suitable alcohol (e.g., a C₁-C₆ alcohol) via transesterification, or with ammonia or a suitable amine (e.g., a mono-C₁-C₆ alkylamine or a di-C₁-C₆ alkylamine) to form an amide. Alternatively, the alkali metal salt of the compound of Formula I can be reacted with an appropriate halide (e.g., a C₁-C₆ alkyl chloride or bromide, (5-methyl-2-oxo-1,3-dioxolen-4-yl)methyl chloride or bromide, neopentyl chloride or bromide, benzofuranone chloride or bromide, [(isopropoxycarbonyl)oxy]methyl chloride or bromide, or the like) to produce pharmaceutically acceptable esters or amides. In a similar manner, when the compound of Formula I contains a hydroxyl group, pharmaceutically acceptable esters can be prepared by conventional condensation reactions with carboxylic acids, acyl chlorides, acid anhydrides, or their active derivatives.

The aforementioned esters may include, for example, C₁-C₆ alkyl esters such as methyl, ethyl, propyl, isopropyl, butyl, sec-butyl, tert-butyl, pentyl, and hexyl esters; C₃-C₆ cycloalkyl esters such as cyclopentyl and cyclohexyl esters; C₆-C₁₀ aryl esters such as phenyl and naphthyl esters; C₆-C₁₀ aryl-C₁-C₆ alkyl esters such as benzyl, phenethyl, α-methylbenzyl, 3-phenylpropyl, 4-phenylbutyl, 6-phenylhexyl, diphenylmethyl, and triphenylmethyl esters; or hydrolyzable esters in vivo such as (5-methyl-2-oxo-1,3-dioxolen-4-yl)methyl ester, neopentyl ester, benzofuranone ester, [(isopropoxycarbonyl)oxy]methyl ester, [(cyclohexyloxycarbonyl)oxy]methyl ester, and 1-[(cyclohexyloxycarbonyl)oxy]ethyl ester.

The aforementioned amides may include, for example, primary amides (-CONH₂); mono-C₁-C₆ alkyl or mono-C₃-C₆ cycloalkyl amides such as N-methylamide, N-ethylamide, N-propylamide, N-isopropylamide, N-butylamide, N-sec-butylamide, N-tert-butylamide, N-pentylamide, N-hexylamide, N-cyclopropylamide, N-cyclopentylamide, and N-cyclohexylamide; or di-C₁-C₆ alkyl, N-C₁-C₆ alkyl-N-C₃-C₆ cycloalkyl, or di-C₃-C₆ cycloalkyl amides such as N,N-dimethylamide, N,N-diethylamide, N,N-dipropylamide, N,N-diisopropylamide, N-methyl-N-ethylamide, N-methyl-N-propylamide, N-methyl-N-butylamide, N-ethyl-N-propylamide, N-ethyl-N-butylamide, N-butyl-N-cyclopentylamide, N-ethyl-N-cyclopropylamide, and N,N-dicyclohexylamide.

The term "solvate" refers to a complex formed between a compound of Formula I and solvent molecules in a defined stoichiometric ratio. The term "hydrate" refers to a complex formed between the compound of the present application and water.

The term "active metabolite" refers to a derivative of the compound that exhibits pharmacological activity upon metabolism of the compound.

The term "polymorph" refers to the compound of the present application existing in different crystalline lattice forms.

The term "isotopically labeled compound" refers to a compound of the present application labeled with one or more isotopes. Isotopes may include, for example, isotopes of H, C, N, O, P, F, and S, such as ²H, ³H, ¹³C, ¹⁴C, ¹⁵N, ¹⁸O, ¹⁷O, ³¹P, ³²P, ³⁵S, ¹⁸F, and ³⁶S.

The term "isomer" refers to compounds having the same molecular formula but differing in the spatial arrangement of atoms. Compounds of the present application may contain asymmetric or chiral centers, double bonds, or other structural elements and may thus include optical isomers, geometric isomers, tautomers, atropisomers, and other stereoisomeric forms. These isomers, including individual stereoisomers and racemates, are encompassed within the scope of the present application. For example, optical isomers can be prepared using chiral resolution, chiral synthesis, chiral reagents, or other conventional techniques to obtain optically active (R)- and (S)-enantiomers, as well as D and L forms. Non-racemic isomers can also be generated by reaction with a suitable chiral reagent (e.g., a chiral alcohol or Mosher's acid chloride), separated, and converted (e.g., by hydrolysis) to the corresponding single stereoisomer. Separation can also be achieved via chromatographic methods.

Neurodegenerative diseases and neural injuries have a severe impact on human health and are often difficult to cure. With the advancement of stem cell biology, neuroscience, and reprogramming technologies, several effective therapeutic approaches have been developed. For example, stem cell replacement therapy can be applied to diseases and injuries of the central and peripheral nervous systems by regenerating affected neural tissues with neurons and other neural cells. One of the main strategies to achieve this goal is to either activate the endogenous regenerative capacity of neural stem cells or transplant neural or embryonic cells to restore normal neural development. Although the use of stem cells in neurological disorders is widespread, significant challenges remain. In particular, controlling the efficient and stable directed differentiation of stem cells continues to be a major focus in the field of stem cell biology.

In view of the foregoing background, in a first aspect, the present application provides an induction medium comprising a BMPR1B inhibitor; the BMPR1B inhibitor has the structure represented by Formula I, or a pharmaceutically acceptable salt, ester, amide, solvate, active metabolite, polymorph, isotopically labeled derivative, isomer, or prodrug thereof; 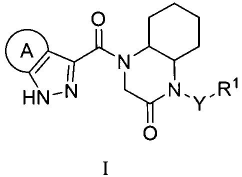 wherein the ring A is selected from any one of the following structures: 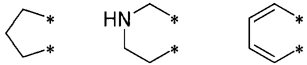
Y is -(CH₂)ₙ-, where n is 0, 1, 2, or 3;
R¹ is selected from -H, -D, unsubstituted or R²-substituted C₁-C₆ alkyl, C₁-C₆ alkoxy, C₆-C₁₀ aryl, or C₃-C₁₀ heteroaryl;
R² is selected from -F, -Cl, -Br, -I, -OH, -COOH, C₁-C₆ alkyl, or C₁-C₆ alkoxy;
"*" represents a point of attachment.

The medium provided in the present application contains a BMPR1B inhibitor, which is a pyrazolecarbonyl piperazinone compound specially designed for its structure. This compound can form a relatively stable complex with BMPR1B through hydrogen bonding, hydrophobic interactions, and other mechanisms, thereby modulating BMPR1B activity. As a result, it can regulate downstream gene expression and physiological processes, enabling pluripotent stem cells to efficiently and directionally differentiate into neural lineage cells. This provides a potential approach for the application of stem cell biology in the treatment of neurodegenerative diseases and neural injuries.

In some embodiments, Y is -(CH₂)ₙ-, where n is 0, 1, or 2; preferably, Y is -(CH₂)ₙ-, where n is 0 or 1; more preferably, Y is -(CH₂)ₙ-, where n is 0 or 1.

In some embodiments, R¹ is selected from -H, -D, unsubstituted or R²-substituted C₁-C₆ alkyl, C₆-C₁₀ aryl, or C₃-C₁₀ heteroaryl. Preferably, R¹ is selected from -H, -D, unsubstituted or R²-substituted C₃-C₆ heteroaryl. More preferably, R¹ is selected from -H, -D, unsubstituted or R²-substituted furyl, thienyl, pyrrolyl, pyrazolyl, imidazolyl, oxazolyl, isoxazolyl, thiazolyl, isothiazolyl, pyranyl, thianyl, pyridyl, diazinyl, or pyrimidinyl. Still more preferably, R¹ is selected from -H, -D, or any one of the following substituents: 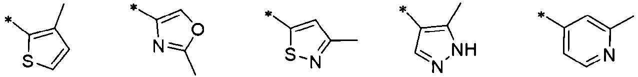 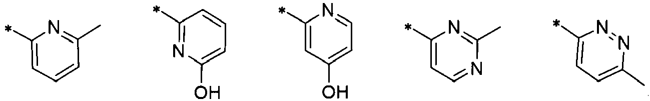 wherein "*" represents a point of attachment.

When R¹ is selected from heteroaryl, particularly heteroaryl substituted with a methyl or hydroxyl group, the resulting pyrazolecarbonyl piperazinone compound exhibits a higher binding energy with the docking site of BMPR1B, while showing lower physiological toxicity. This property is favorable for enhancing both the pharmacological efficacy and biocompatibility of pyrazolecarbonyl piperazinone compounds as BMPR1B inhibitors.

In some embodiments, R² is selected from -OH or C₁-C₆ alkyl. Preferably, R² is selected from -OH or C₁-C₄ alkyl. More preferably, R² is selected from -OH, methyl, ethyl, n-propyl, or isopropyl.

In some embodiments, the BMPR1B inhibitor has a structure represented by any one of Formulas I-1 to I-13, or a pharmaceutically acceptable salt, ester, amide, solvate, active metabolite, polymorph, isotopically labeled compound, isomer, or prodrug thereof;

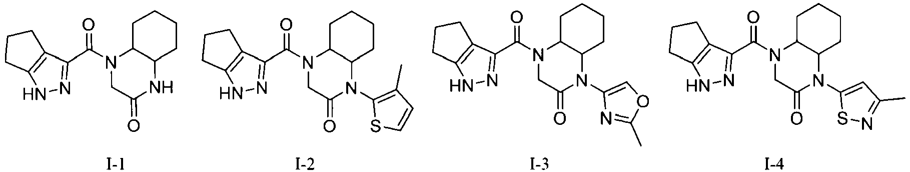

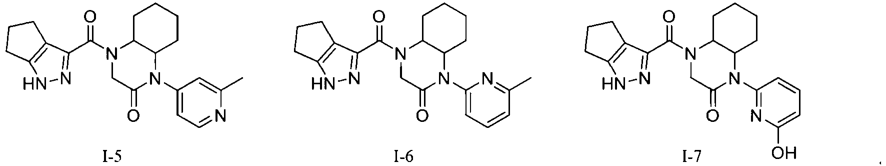

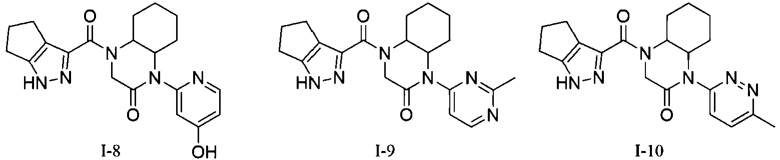

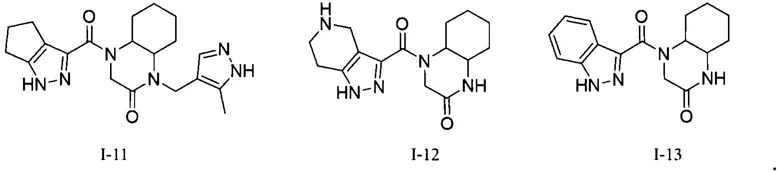

In some embodiments, the BMPR1B inhibitor has a structure represented by Formula II, or a pharmaceutically acceptable salt, ester, amide, solvate, active metabolite, polymorph, isotopically labeled compound, isomer, or prodrug thereof; 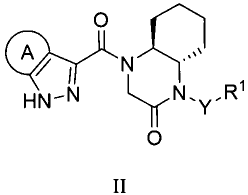 wherein ring A, Y, and R¹ are as defined in any of the foregoing embodiments.

In some embodiments, the BMPR1B inhibitor has a structure represented by any one of Formulas II-1 to II-13, or a pharmaceutically acceptable salt, ester, amide, solvate, active metabolite, polymorph, isotopically labeled compound, isomer, or prodrug thereof;

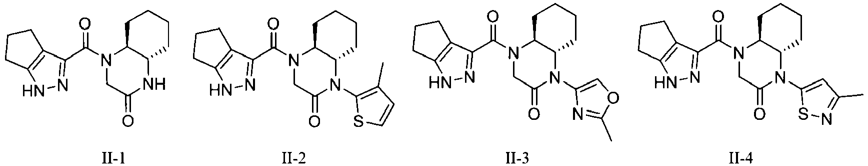

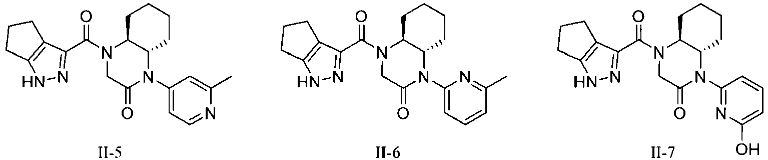

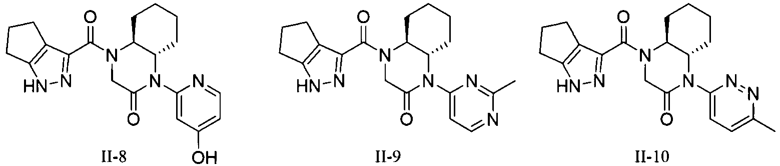

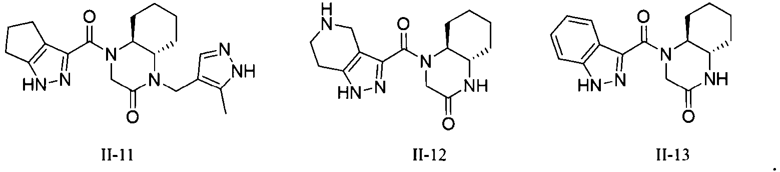

In some embodiments, the concentration of the BMPR1B inhibitor is 1 µM to 50 µM. Preferably, the concentration of the BMPR1B inhibitor is 1 µM to 20 µM, and more preferably, 1 µM to 10 µM. Optionally, the concentration of the BMPR1B inhibitor may be, for example, 2.5 µM, 5 µM, 7.5 µM, 10 µM, 12.5 µM, 15 µM, 17.5 µM, 20 µM, 25 µM, 30 µM, 35 uM, 40 µM, or 45 µM. A suitable concentration of the BMPR1B inhibitor can more efficiently promote the directed differentiation of pluripotent stem cells into neural lineage cells without causing damage.

In some embodiments, the induction medium further comprises one or more of a basal medium, growth factors, inorganic salts, and additives. The growth factors may include one or more of vitamins, progesterone, 1,4-butanediamine, and insulin. The additives may include one or more of piperidinol oxide, luteolin, D(+)-galactose, and recombinant human transferrin.

In some embodiments, the induction medium satisfies one or more of the following conditions:
(1) the basal medium is DMEM medium or DMEM-F12 medium, and the basal medium is supplemented with minimum essential medium non-essential amino acids;
(2) the vitamins comprise one or more of vitamin E, vitamin B12, and vitamin C;
(3) the inorganic salts comprise one or more of sodium chloride and sodium selenite.

In some embodiments, the induction medium satisfies one or more of the following conditions:
(1) the growth factors comprise vitamins, 2.0 ng/mL to 15 ng/mL progesterone, 2 µg/mL to 50 µg/mL 1,4-butanediamine, and 5 mg/L to 40 mg/L insulin, and the vitamins comprise 0.1 µg/mL to 15 µg/mL vitamin E, 0.5 µM to 2.4 µM vitamin B12, and 32 mg/L to 128 mg/L vitamin C;
(2) the inorganic salts comprise 0.5 g/L sodium chloride and 13.6 µg/L sodium selenite;
(3) the additives comprise 10 µM to 200 µM piperidinol oxide, 5 µM to 150 µM luteolin, 5 µg/mL to 25 µg/mL D(+)-galactose, and 50 ng/mL to 200 ng/mL recombinant human transferrin.

In some embodiments, the induction medium satisfies one or more of the following conditions:
(1) The growth factors include vitamins, 6.3 ng/mL progesterone, 23 µg/mL 1,4-butanediamine, and 22 mg/L insulin; the vitamins include 1 µg/mL vitamin E, 1.2 µM vitamin B12, and 64 mg/L vitamin C;
(2) The inorganic salts include 0.5 g/L sodium chloride and 13.6 µg/L sodium selenite;
(3) The additives include 50 µM piperidinol oxide, 60 µM luteolin, 12.5 µg/mL D(+)-galactose, and 100 ng/mL recombinant human transferrin.

In a second aspect, the present application provides a method for inducing the differentiation of pluripotent stem cells into neural lineage cells, comprising the following step:
adherently culturing pluripotent stem cells in the induction medium according to any of the above embodiments to obtain neural lineage cells.

In some embodiments, NANOG is used as a marker to identify pluripotent stem cells, and PAX6 is used as a marker to identify neural lineage cells. Since NANOG is expressed only in pluripotent stem cells and PAX6 only in neural lineage cells, selecting these markers allows quantitative tracking of the differentiation process from pluripotent stem cells to neural lineage cells and verifies the induction outcome.

In some embodiments, adherent culture is performed in the presence of a basement membrane preparation. Preferably, the basement membrane preparation includes one or more of Matrigel, laminin, and fibronectin.

In some embodiments, the pluripotent stem cells are mammalian pluripotent stem cells. Optionally, the pluripotent stem cells are human pluripotent stem cells, which may include, for example, H9 or H1 cell lines, or human induced pluripotent stem cells (e.g., obtained by reprogramming CD34+ cells as described in CN109628383B).

In some embodiments, the neural lineage cells include one or more of neural progenitor cells, neurons, astrocytes, and oligodendrocytes.

In a third aspect, the present application provides the use of the BMPR1B inhibitor mentioned in any of the above embodiments of the induction medium for inducing the differentiation of pluripotent stem cells into neural lineage cells.

### Compound Synthesis:

In the following, if there is a discrepancy between the compound name and the structural formula, the structural formula shall prevail.

### Synthetic Route A

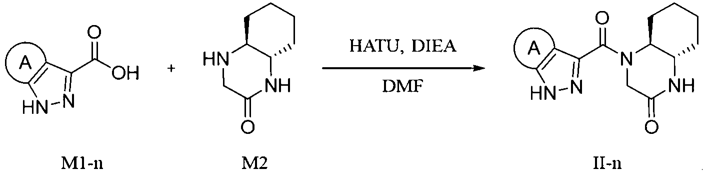

Intermediate M1-n (0.12 mmol), (4aS,8aS)-tetrahydroquinoline-2(1H)-one (M2, 15 mg, 0.1 mmol), and 2-(7-azabenzotriazol)-N,N,N',N'-tetramethyluronium hexafluorophosphate (HATU, 380 mg, 1 mmol) were mixed, then N,N-diisopropylethylamine (DIEA, 2 mL) and N,N-dimethylformamide (DMF, 20 mL) were added. The reaction mixture was stirred at room temperature for 18 hours. Ethyl acetate and water were added to the reaction mixture, the organic layer was separated, dried over sodium sulfate, and concentrated under reduced pressure. The residue was purified by silica gel column chromatography (ethyl acetate : hexane = 3:7) to obtain the target product II-n.

Compounds II-1 and II-13 were obtained via Synthetic Route A.
II-1:
   (4aS,8aS)-4-(1,4,5,6-tetrahydrocyclopenta[c]pyrazole-3-carbonyl)octahydroquinoxalin-2(1H)-one (12 mg, 41%), LC- MS m/z=289.2[M+H]⁺_{∘}
II-13:
   (4aS,8aS)-4-(1H-indazole-3-carbonyl)octahydroquinoxalin-2(1H)-one (17 mg, 57%), LC- MS m/z=299.2 [M+H]⁺_{∘}

### Synthetic Route B

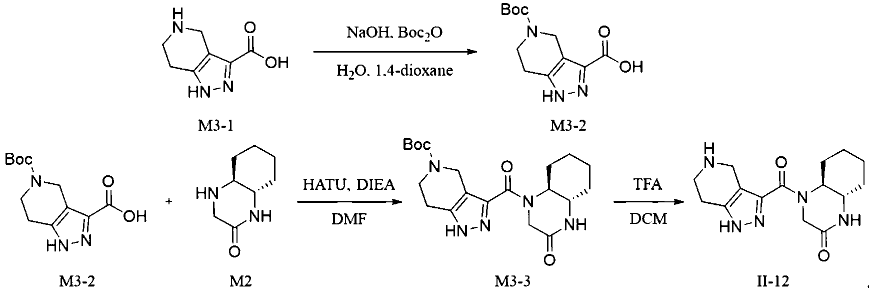

Compound II-12 was obtained via Synthetic Route B.
II-12:
(4aS,8aS)-4-(4,5,6,7-tetrahydro-1H-pyrazolo[4,3-c]pyridine-3-carbonyl)octahydroquinoxalin-2(1H)-one
(1) At 0°C, a solution of 4,5,6,7-tetrahydro-2H-pyrazolo[4,3-c]pyridine-3-carboxylic acid (M3-1, 0.167 g, 1 mmol) in 2 mL of 1 mol/L NaOH and 0.6 mL of 1,4-dioxane was added dropwise with a solution of di-tert-butyl dicarbonate (Boc₂O, 0.25 g, 1.1 mmol) in 1.5 mL of 1,4-dioxane. The reaction mixture was stirred at 0°C for 0.5 h and then allowed to react overnight at room temperature, maintaining pH 8-9. After the reaction, the mixture was diluted with 5 mL of H₂O and extracted with n-hexane. The aqueous phase was acidified to pH 2-3 with citric acid and extracted with ethyl acetate. The organic phase was washed with saturated brine, dried over anhydrous MgSO₄, and concentrated under reduced pressure to afford 5-(tert-butoxycarbonyl)-4,5,6,7-tetrahydro-2H-pyrazolo[4,3-c]pyridine-3-carboxylic acid (M3-2, 200 mg, 75%), LC-MS m/z = 268.1 [M+H]⁺.
(2) M3-2 (20 mg, 0.075 mmol) was mixed with (4aS,8aS)-octahydroquinoline-2(1H)-one (M2, 15 mg, 0.1 mmol) and 2-(7-azabenzotriazol)-N,N,N',N'-tetramethyluronium hexafluorophosphate (HATU, 38 mg, 0.1 mmol), followed by addition of N,N-diisopropylethylamine (DIEA, 0.2 mL) and N,N-dimethylformamide (DMF, 2 mL). The mixture was stirred at room temperature for 18 hours. Ethyl acetate and water were added, and the organic layer was separated, dried over sodium sulfate, and concentrated under reduced pressure. The residue was purified by silica gel column chromatography (ethyl acetate : hexane = 3:7) to give tert-butyl 3-((4aS,8aS)-3-oxodecahydroquinoline-1-carbonyl)-1,4,6,7-tetrahydro-SH-pyrazolo[4,3-c]pyridine-5-carboxylate (M3-3, 18 mg, 60%), LC-MS m/z = 404.2 [M+H]⁺.
(3) M3-3 (18 mg, 0.04 mmol) and trifluoroacetic acid (TFA, 35 mg, 0.3 mmol) were added to 2 mL of dichloromethane (DCM) and stirred at room temperature for 30 minutes. Ethyl acetate and water were then added, and the organic layer was separated, dried over sodium sulfate, and concentrated under reduced pressure to afford II-12 (13 mg, 96%), LC-MS m/z = 304.2 [M+H]⁺.

### Synthetic Route C

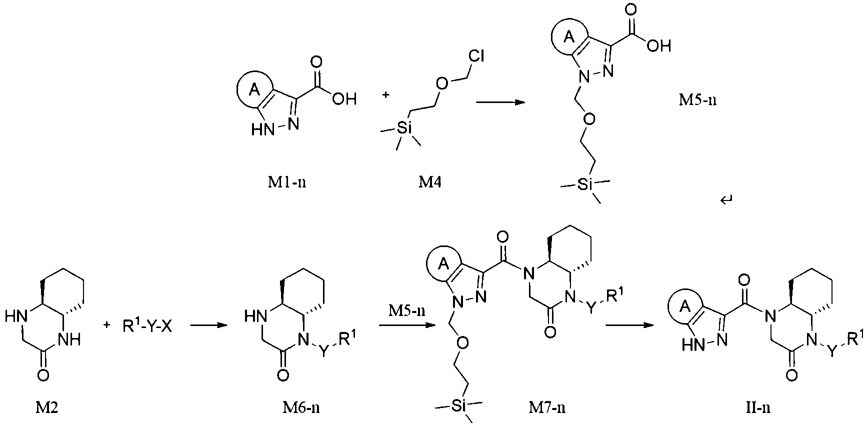

(1) Sodium hydride (60% dispersion, 1.07 g, 26.9 mmol) dissolved in 5 mL of tetrahydrofuran (THF) was cooled to 0 °C. A solution of M1-n (21.5 mmol) in 50 mL of THF was added dropwise over 15 minutes, and the mixture was stirred at 0 °C for 1 hour. A solution of (2-(chloromethoxy)ethyl)trimethylsilane (M4, 4.76 mL, 26.9 mmol) in 50 mL of THF was then added dropwise, and the mixture was stirred at room temperature for 48 hours. Water and ethyl acetate were slowly added to the reaction mixture. The organic layer was separated, dried over sodium sulfate, and concentrated under reduced pressure to afford M5-n.
(2) In a 20 mL reaction vessel, R¹-Y-X (0.698 mmol, where X represents a halogen, and R¹ and are as defined above), (4aS,8aS)-octahydroquinoline-2(1H)-one (M2, 108 mg, 0.698 mmol, 1.0 eq.), cesium carbonate (796 mg, 2.443 mmol, 2.5 eq.), 4,5-bis(diphenylphosphino)-9,9-dimethylxanthene (20 mg, 0.035 mmol, 0.05 eq.) and palladium acetate (7.84 mg, 0.035 mmol, 0.05 eq.) were dissolved in 3 mL of THF and purged with nitrogen for 10 minutes. The reaction was heated to 70 °C and maintained for 90 minutes. The mixture was filtered, washed with dichloromethane, and concentrated. The residue was purified by silica gel column chromatography (0-10% ethyl acetate/dichloromethane) to afford M6-n.
(3) M6-n (1.0 mmol, 1.0 eq.) was dissolved in 10 mL of dichloromethane, followed by addition of M5-n (1.2 mmol, 1.2 eq.), triethylamine (200 mg, 2.0 mmol, 2.0 eq.) and 2-(7-azabenzotriazol)-N,N,N',N'-tetramethyluronium hexafluorophosphate (570 mg, 1.5 mmol, 1.5 eq.). The mixture was stirred overnight. The reaction was monitored by thin-layer chromatography, and upon disappearance of the starting material, the mixture was diluted with dichloromethane, washed with brine, dried over sodium sulfate, and concentrated under reduced pressure. The residue was purified by silica gel column chromatography (petroleum ether : ethyl acetate = 2:1) to afford M7-n.
(4) At 5 °C, triethylsilane (2.30 g, 19.8 mmol) was added to a solution of M7-n (3.95 mmol) in 39.5 mL of THF. The mixture was stirred at room temperature for 3 hours, concentrated, washed with brine, dried over sodium sulfate, and concentrated under reduced pressure. The residue was purified by silica gel column chromatography (ethyl acetate : petroleum ether = 20-100%) to afford the target product II-n.

Compounds II-2 to II-10 were obtained via Synthetic Route C.
II-2:
   (4aS,8aS)- 1-(3-methylthiophen-2-yl)-4-(1,4,5,6-tetrahydrocyclopenta[c]pyrazole-3-carbonyl)octahydroquinoxalin-2(1H)-one (1504 mg, 99%), LC-MS m/z=384.2 [M+H]⁺_{∘}
II-3:
   (4aS,8aS)-1-(2-methyloxazol-4-yl)-4-(1,4,5,6-tetrahydrocyclopenta[c]pyrazole-3-carbonyl)octahydroquinoxalin-2(1H)-one (1445 mg, 99%), LC-MS m/z=370.2 [M+H]⁺_{∘}
II-4:
   (4aS,8aS)-1-(3-methylisothiazol-5-yl)-4-(1,4,5,6-tetrahydrocyclopenta[c]pyrazole-3-carbonyl)octahydroquinoxalin-2(1H)-one (1507 mg, 99%), LC-MS m/z=386.2 [M+H]⁺_{∘}
II-5:
   (4aS,8aS)-1-(2-methylpyridin-4-yl)-4-(1,4,5,6-tetrahydrocyclopenta[c]pyrazole-3-carbonyl)octahydroquinoxalin-2(1H)-one (1469 mg, 98%), LC-MS m/z=380.2 [M+H]⁺_{∘}
II-6:
   (4aS,8aS)-1-(6-methylpyridin-2-yl)-4-(1,4,5,6-tetrahydrocyclopenta[c]pyrazole-3-carbonyl)octahydroquinoxalin-2(1H)-one (1484 mg, 99%), LC-MS m/z=380.2 [M+H]⁺_{∘}
II-7:
   (4aS,8aS)-1-(6-hydroxypyridin-2-yl)-4-(1,4,5,6-tetrahydrocyclopenta[c]pyrazole-3-carbonyl)octahydroquinoxalin-2(1H)-one (1491 mg, 99%), LC-MS m/z=382.2 [M+H]⁺_{∘}
II-8:
   (4aS,8aS)-1-(4-hydroxypyridin-2-yl)-4-(1,4,5,6-tetrahydrocyclopenta[c]pyrazole-3-carbonyl)octahydroquinoxalin-2(1H)-one (1491 mg, 99%), LC-MS m/z=382.2 [M+H]⁺_{∘}
II-9:
   (4aS,8aS)-1-(2-methylpyrimidin-4-yl)-4-(1,4,5,6-tetrahydrocyclopenta[c]pyrazole-3-carbonyl)octahydroquinoxalin-2(1H)-one (1488 mg, 99%), LC-MS m/z=381.2 [M+H]⁺_{∘}
II-10:
   (4aS,8aS)-1-(6-methylpyridazin-3-yl)-4-(1,4,5,6-tetrahydrocyclopenta[c]pyrazole-3-carbonyl)octahydroquinoxalin-2(1H)-one (1413 mg, 94%), LC-MS m/z=381.2 [M+H]⁺_{∘}

### Synthetic Route D

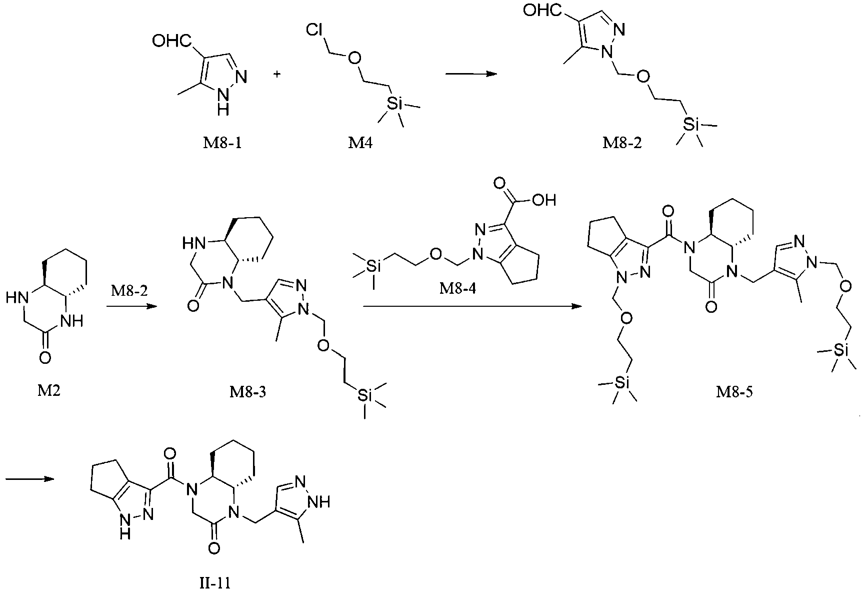

Compound II-11 was obtained via Synthetic Route D.
II-11:
(4aS,8aS)-1-((5-methyl-1H-pyrazol-4-yl)methyl)-4-(1,4,5,6-tetrahydrocyclopenta[c]pyrazole-3-carbonyl)octahydroquinoxalin-2(1H)-one
(1) Sodium hydride (60% dispersion in THF, 1.07 g, 26.9 mmol) dissolved in 5 mL of tetrahydrofuran (THF) was cooled to 0 °C. A solution of 5-methyl-1H-pyrazole-4-carbaldehyde (M8-1, 2.37 g, 21.5 mmol) in 50 mL of THF was added dropwise over 15 minutes, and the mixture was stirred at 0 °C for 1 hour. A solution of (2-(chloromethoxy)ethyl)trimethylsilane (M4, 4.76 mL, 26.9 mmol) in 50 mL of THF was added dropwise, and the mixture was stirred at room temperature for 48 hours. Water and ethyl acetate were slowly added, and the organic layer was separated, dried over sodium sulfate, and concentrated under reduced pressure to afford 5-methyl-1-((2-(trimethylsilyl)ethoxy)methyl)-1H-pyrazole-4-carbaldehyde (M8-2, 4.96 g, 96%). LC-MS m/z = 240.1 [M+H]⁺.
(2) In a 20 mL reaction vessel, M8-2 (168 mg, 0.698 mmol), (4aS,8aS)-octahydroquinoline-2(1H)-one (M2, 108 mg, 0.698 mmol, 1.0 eq.), cesium carbonate (796 mg, 2.443 mmol, 2.5 eq.), 4,5-bis(diphenylphosphino)-9,9-dimethylxanthene (20 mg, 0.035 mmol, 0.05 eq.) and palladium acetate (7.84 mg, 0.035 mmol, 0.05 eq.) were dissolved in 3 mL of THF and purged with nitrogen for 10 minutes. The reaction was heated to 70 °C and maintained for 90 minutes. The mixture was filtered, washed with dichloromethane, and concentrated. The residue was purified by silica gel column chromatography (0-10% ethyl acetate/dichloromethane) to give (4aS,8aS)-1-((5-methyl-1-((2-(trimethylsilyl)ethoxy)methyl)-1H-pyrazol-4-yl)methyl)octahydroquinoline-2(1H)-one (M8-3, 244 mg, 92.2%). LC-MS m/z = 378.3 [M+H]⁺.
(3) M8-3 (379 mg, 1.0 mmol, 1.0 eq.) was dissolved in 10 mL of dichloromethane, followed by addition of 1-((2-(trimethylsilyl)ethoxy)methyl)-1,4,5,6-tetrahydrocyclopenta[c]pyrazole-3-carboxylic acid (M8-4, 339 mg, 1.2 mmol, 1.2 eq.), triethylamine (200 mg, 2.0 mmol, 2.0 eq.) and 2-(7-azabenzotriazol)-N,N,N',N'-tetramethyluronium hexafluorophosphate (570 mg, 1.5 mmol, 1.5 eq.). The mixture was stirred overnight. The reaction was monitored by thin-layer chromatography, and upon disappearance of the starting material, the mixture was diluted with dichloromethane, washed with brine, dried over sodium sulfate, and concentrated under reduced pressure. The residue was purified by silica gel column chromatography (petroleum ether:ethyl acetate = 2:1) to afford (4aS,8aS)-1-((5-methyl-1-((2-(trimethylsilyl)ethoxy)methyl)-1H-pyrazol-4-yl)methyl)-4-(1-((2-(trimethylsilyl)ethoxy)methyl)-1,4,5,6-tetrahydrocyclopenta[c]pyrazole-3-carbonyl)octahydroquinoxalin-2(1H)-one (M8-5, 424 mg, 66%). LC-MS m/z = 642.4 [M+H]⁺.
(4) At 5 °C, triethylsilane (2.30 g, 19.8 mmol) was added to a solution of M8-5 (2540 mg, 3.95 mmol) in 39.5 mL of THF. The mixture was stirred at room temperature for 3 hours, concentrated, washed with brine, dried over sodium sulfate, and concentrated under reduced pressure. The residue was purified by silica gel column chromatography (ethyl acetate : petroleum ether = 20-100%) to afford II-11 (1510 mg, 99%). LC-MS m/z = 383.2 [M+H]⁺.

### Example 1

Molecular docking of the pyrazolecarbonyl piperazinone compounds of the present application with the BMPR1B target protein was performed using AutoDock Vina software, generating 13 docking conformations for each compound. The binding energy of the optimal docking conformation between each compound and BMPR1B was calculated. The docking results are shown in Table 1. Column 2 of Table 1 represents the binding free energy calculated by AutoDock Vina; the more negative the value, the stronger the compound binds to the target protein. Predicted values show that the absolute values of the binding energies of the pyrazolecarbonyl piperazinone compounds are all significantly greater than the threshold value of 3 set according to the target features.

Cytotoxicity prediction for the pyrazolecarbonyl piperazinone compounds was performed using eToxPred software. The cytotoxicity results for each compound are shown in Column 3 of Table 1. Predicted values indicate that all compounds have toxicity below the threshold of 0.58 (values above 0.58 suggest potential toxicity).

**Table 1. Molecular Docking Binding Energies and Predicted Cytotoxicity of Pyrazolecarbonyl Piperazinone Compounds**

| Compound Number | Molecular Docking Binding Energy (kcal/mol) | Predicted Cytotoxicity |
|---|---|---|
| II-1 | -9.5 | 0.54 |
| II-2 | -9.9 | 0.48 |
| II-3 | -10.6 | 0.50 |
| II-4 | -10.1 | 0.48 |
| II-5 | -11.8 | 0.49 |
| II-6 | -11.7 | 0.48 |
| II-7 | -12 | 0.47 |
| II-8 | -11.9 | 0.52 |
| II-9 | -11.4 | 0.46 |
| II-10 | -11.7 | 0.49 |
| II-11 | -10.2 | 0.47 |
| II-12 | -8.9 | 0.52 |
| II-13 | -10.2 | 0.57 |

### Example 2

A total of 330 kinase candidates (15-50 nM, 2.5 µL) were mixed with compound II-1 (2.5 mM, 25 nL, prepared in DMSO) and incubated at 25 °C for 10 minutes. Then, a mixture of kinase peptide substrate (0.2 mg/mL; supplier: GenScript) and ATP (10-60 µM; supplier: Promega; product number: V915B) was added (total 2.5 µL, prepared in assay buffer), and the reaction was carried out at 25 °C for 60-120 minutes. Finally, the reaction products were quantified using HTRF1 or ADP-Glo2 methods. During the kinase activity assay, the consumption of each substrate was kept below 10%, and each kinase assay was performed in duplicate.

The results showed that II-1 exhibited significant inhibitory activity against BMPR1B kinase (Figure 1A; note: all test results are displayed, but for clarity, only one kinase name is shown on the x-axis for every 10 kinases). At the same concentration, II-1 did not inhibit the kinase activity of TGFBR1 or BMPR2. Furthermore, at different concentrations (up to 12.5 µM, threefold serial dilution), II-1 still showed no inhibition of TGFBR1 or BMPR2 kinase (Figure 1B, C). These results confirm the selective binding ability of II-1 to its target.

To examine the above results from a structural perspective, PyMol software (supplier: Schrödinger) was used to analyze the complexes of compounds II-1, II-2, II-11, and II-13 with BMPR1B. As shown in Figure 2, the complex structures were obtained by molecular docking. The N- and C-terminal domains of BMPR1B are represented as cartoon models of different shapes. The loop structures of BMPR1B, including the activation loop, are represented as cartoon models with different shades of gray. The pyrazolecarbonyl piperazinone compounds are shown as stick-and-ring models, the interacting amino acid backbones/side chains are shown as stick models, hydrogen bonds are represented as solid lines, and hydrophobic interactions are represented as dashed lines. These results also indicate that pyrazolecarbonyl piperazinone compounds have structural features consistent with binding to BMPR1B.

### Example 3

The inhibitory effect of the pyrazolecarbonyl piperazinone compounds on the BMP signaling pathway was further validated by Western blot analysis. Commercial human pluripotent stem cell line H1 was seeded in a 6-well plate at 1.5 × 10^6 cells per well in 2 mL of culture medium. After one day of culture, the medium was replaced, and 6 µL of 30 µM pyrazolecarbonyl piperazinone compound II-1 was added. Cells were treated for 12, 24, and 36 hours, after which the cells were harvested and protein samples were prepared according to the instructions of a commercial kit (Beyotime Biotechnology; Cat. No. P0013). Western blot analysis was performed as described in the literature (H. Zhang et al., 2022; N. Zhang et al., 2021). The results showed that within 24 hours of treatment, pyrazolecarbonyl piperazinone compound II-1 did not inhibit the phosphorylation of the downstream TGF signaling pathway protein SMAD2/3, whereas the phosphorylation levels of the downstream BMP signaling pathway proteins SMAD1/5/8 were significantly suppressed (Figure 3). These results further demonstrate that pyrazolecarbonyl piperazinone compound II-1 selectively inhibits the BMP signaling pathway, consistent with the kinase activity assay results (Figures 1B and 1C).

**Table 2. Antibody Information**

| Antibody Name | Catalog Number | Source |
|---|---|---|
| SMAD1 antibody | ab33902 | Abcam |
| P-SMAD1 antibody | ab226821 | Abcam |
| SMAD2 antibody | ab40855 | Abcam |
| P-SMAD2 antibody | ab188334 | Abcam |
| β-Actin antibody | ab8226 | Abcam |
| HRP-conjugated Affinipure Goat Anti-Mouse IgG(H+L) | ab205719 | Abcam |
| HRP-conjugated Affinipure Goat Anti-Rabbit IgG(H+L) | ab6721 | Abcam |

### Example 4: Neural Lineage Induction and Identification

### (1) Medium Formulation

The induction medium comprises Dulbecco's Modified Eagle Medium (DMEM), Minimum Essential Medium Non-Essential Amino Acids (MEM NEAA, Cat. No. 11140076, Thermo Fisher), 4-Hydroxy-TEMPO (50 µM), D(+)-galactose (12.5 µg/mL), vitamin E (1 µg/mL), vitamin B12 (1.2 µM), vitamin C (64 mg/L), progesterone (6.3 ng/mL), 1,4-butanediamine (23 µg/mL), luteolin (60 µM), sodium chloride (0.5 g/L), sodium arsenite (13.6 µg/L), insulin (22 µg/mL), recombinant human transferrin (100 ng/mL), and the BMPR1B inhibitor of the present invention (induction medium A uses BMPR1B inhibitor II-1; induction medium B uses BMPR1B inhibitor II-13).

### (2) Pluripotent Stem Cell Expansion and Passaging

Commercial human pluripotent stem cell lines H1, H9, and induced pluripotent stem cells (iPSCs) were used. The iPSCs were prepared according to the "reprogramming medium for the culture of reprogrammed iPSCs" using CD34+ cells (CN109628383A, Example 2). T25 culture flasks were coated with Matrigel (STEMCELL Technologies) and incubated at 37°C for at least one hour. Cells were seeded at 1 × 10^6 per T25 flask for expansion and passaging.

### (3) Neural Induction

6-well plates were coated with 50 µg/mL poly-L-lysine (SIGMA, Cat. No. P6407) and incubated at 37°C for at least 3 hours, followed by coating with 5 µg/mL laminin (SIGMA ALDRICH, Cat. No. 12020) and further incubation at 37°C for at least 3 hours. When pluripotent stem cells reached 70% confluency, they were digested with EDTA at 37°C for 5 minutes, and digestion was terminated using DMEM. Cells were washed, centrifuged, and reseeded in T25 flasks at 2 × 10^5 cells per flask. Induction was carried out using the aforementioned induction media: H9 cells were induced with induction medium A, while H1 cells and iPSCs were induced with induction medium B. The medium was refreshed daily until day 14.

### (4) Sequencing and Data Analysis

Samples were collected on day 7 and day 14 of induction. RNA was processed using the VAHTS Universal V8 RNA-seq Library Prep Kit for Illumina (Vazyme, NR605-01/02), and sequencing was performed on an Illumina platform. Raw sequencing data were processed sequentially with FASTP, HISAT2, and FeatureCounts to obtain gene expression levels, normalized against GAPDH to account for batch differences.

Analysis revealed that with increasing concentrations of BMPR1B inhibitor II-1, the pluripotent stem cell H9 treated with II-1 showed a dose-dependent decrease in the pluripotency marker NANOG (P < 0.05, t-test) and a corresponding increase in the neural progenitor marker PAX6 (P < 0.05, t-test), indicating a dose-dependent effect of II-1 during induction. From day 7 to day 14, NANOG expression decreased while PAX6 expression increased (Figure 3, error bars represent standard deviations of three biological replicates). These results indicate that BMPR1B inhibitor II-1 promotes differentiation of pluripotent stem cells into neural progenitor cells (Figures 4B, D).

Similarly, with increasing concentrations of BMPR1B inhibitor II-13, the neural marker gene MAP2 (A. Caceres, Developmental Brain Research, Vol. 13, Issue 2, April 1984, pp. 314-318) and the glial progenitor marker gene Sox9 (Neyrinck, K et al., Stem Cell Rev and Rep 17, 185521) in H1 cells showed a dose-dependent response to II-13 (Figure 5).

Furthermore, compared to untreated control iPSCs, induction with BMPR1B inhibitor II-13 resulted in significantly increased expression of neural progenitor markers Sox1 and Sox5 (Elkouris M et al., Stem Cells, 2011; 29(1): 89-98; Tina Lai et al., Neuron, 2008; 57(2): 232-247) and neural differentiation marker VIM (Sofia Duarte et al., Nature Communications, vol. 10, 4200, 2019), also exhibiting a dose-dependent effect (Figure 6). These results demonstrate that BMPR1B inhibitors provided herein not only promote differentiation of pluripotent stem cells into neural progenitor cells but also facilitate the formation and differentiation of multiple neural cell types.

The technical features described in the above examples can be combined in any manner. For the sake of brevity, not all possible combinations of the technical features in the above examples have been explicitly described. However, as long as the combination of these technical features does not result in a contradiction, it should be regarded as falling within the scope disclosed in this specification.

The above examples merely illustrate several embodiments of the present application in a specific and detailed manner, and should not be construed as limiting the scope of the invention. It should be noted that those skilled in the art, within the framework of the present application, may make various modifications and improvements, all of which are considered to fall within the protection scope of the present application. Therefore, the protection scope of the present patent application shall be defined by the appended claims, with the description and drawings serving to explain the content of the claims.

## Claims

1. An induction medium comprising a BMPR1B inhibitor, wherein the BMPR1B inhibitor has a structure represented by Formula I, or a pharmaceutically acceptable salt, ester, amide, solvate, active metabolite, polymorph, isotopically labeled compound, isomer, or prodrug thereof; 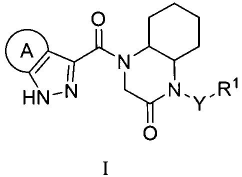 wherein the ring A is selected from any one of the following structures: 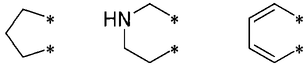
Y is -(CH₂)ₙ-, where n is 0, 1, 2, or 3;
R¹ is selected from -H, -D, unsubstituted or R²-substituted C₁-C₆ alkyl, C₁-C₆ alkoxy, C₆-C₁₀ aryl, or C₃-C₁₀ heteroaryl;
R² is selected from -F, -Cl, -Br, -I, -OH, -COOH, C₁-C₆ alkyl, or C₁-C₆ alkoxy;
"*" represents a point of attachment.

2. The induction medium according to claim 1, wherein the BMPR1B inhibitor satisfies one or more of the following conditions:
(1) Y is -(CH₂)ₙ-, where n is 0, 1, or 2;
(2) R¹ is selected from -H, -D, unsubstituted or R²-substituted C₁-C₆ alkyl, C₆-C₁₀ aryl, or C₃-C₁₀ heteroaryl;
(3) R² is selected from -OH or C₁-C₆ alkyl.

3. The induction medium according to claim 2, wherein the BMPR1B inhibitor satisfies one or more of the following conditions:
(1) Y is -(CH₂)ₙ-, where n is 0 or 1;
(2) R¹ is selected from -H, -D, unsubstituted or R²-substituted C₃-C₆ heteroaryl;
(3) R² is selected from -OH or C₁-C₄ alkyl.

4. The induction medium according to claim 3, wherein the BMPR1B inhibitor satisfies one or more of the following conditions:
(1) Y is -(CH₂)ₙ-, where n is 0 or 1;
(2) R¹ is selected from -H, -D, unsubstituted or R²-substituted furyl, thienyl, pyrrolyl, pyrazolyl, imidazolyl, oxazolyl, isoxazolyl, thiazolyl, isothiazolyl, pyranyl, thianyl, pyridyl, diazinyl, or pyrimidinyl;
(3) R² is selected from -OH, methyl, ethyl, n-propyl, or isopropyl.

5. The induction medium according to any one of claims 1 to 4, wherein R¹ is selected from -H, -D, or any one of the following substituents: 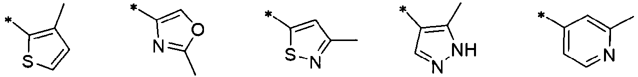 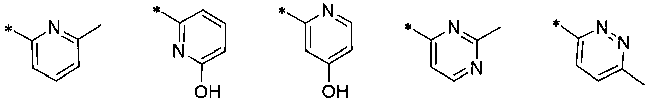 wherein "*" represents a point of attachment.

6. The induction medium according to any one of claims 1 to 4, wherein the BMPR1B inhibitor has a structure represented by any one of Formulas I-1 to I-13, or a pharmaceutically acceptable salt, ester, amide, solvate, active metabolite, polymorph, isotopically labeled compound, isomer, or prodrug thereof;

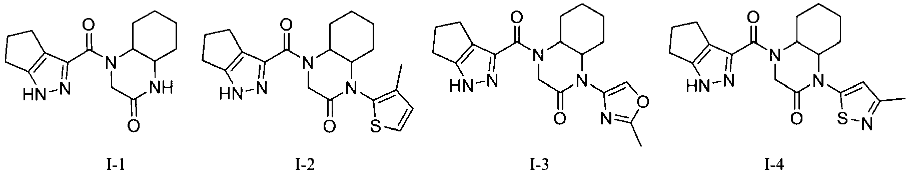

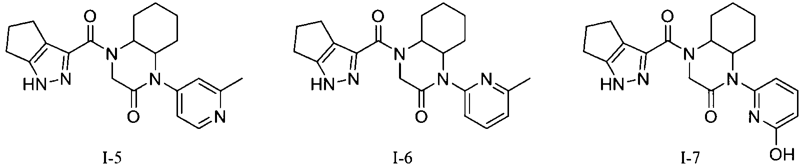

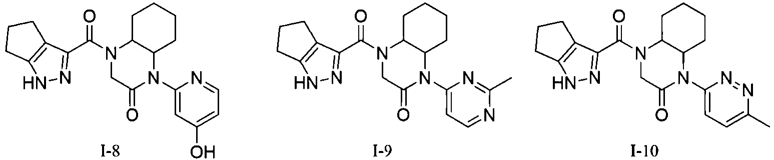

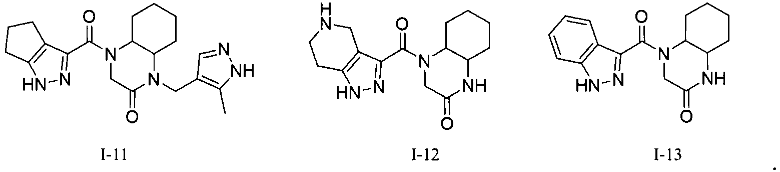

7. The induction medium according to any one of claims 1 to 4, wherein the BMPR1B inhibitor has a structure represented by Formula II, or a pharmaceutically acceptable salt, ester, amide, solvate, active metabolite, polymorph, isotopically labeled compound, isomer, or prodrug thereof; 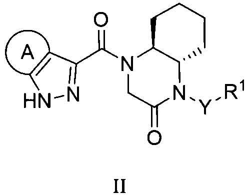 wherein ring A, Y, and R¹ are as defined in any one of claims 1 to 4.

8. The induction medium according to any one of claims 1 to 4, wherein the BMPR1B inhibitor has a structure represented by any one of Formulas II-1 to II-13, or a pharmaceutically acceptable salt, ester, amide, solvate, active metabolite, polymorph, isotopically labeled compound, isomer, or prodrug thereof;

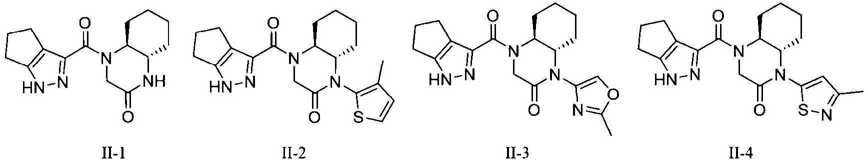

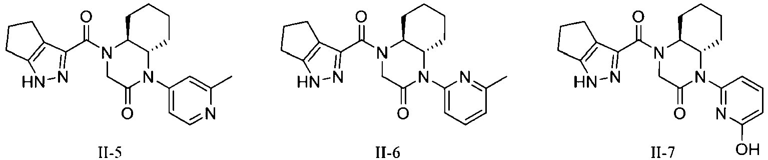

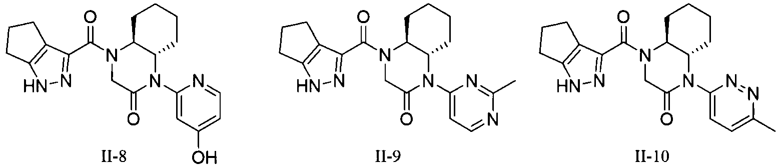

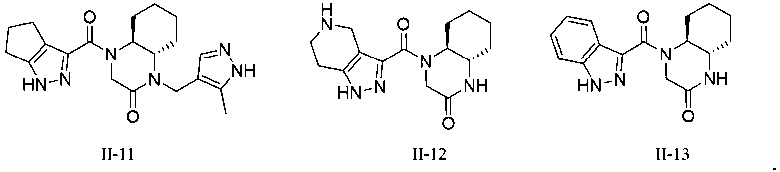

9. The induction medium according to any one of claims 1 to 4, wherein the concentration of the BMPR1B inhibitor is 1 µM to 50 µM.

10. The induction medium according to any one of claims 1 to 4, wherein the induction medium further comprises one or more of a basal medium, growth factors, inorganic salts, and additives;
wherein the growth factors comprise one or more of vitamins, progesterone, 1,4-butanediamine, and insulin;
the additives comprise one or more of piperidinol oxide, luteolin, D(+)-galactose, and recombinant human transferrin.

11. The induction medium according to claim 10, wherein the induction medium satisfies one or more of the following conditions:
(1) the basal medium is DMEM medium or DMEM-F12 medium, and the basal medium is supplemented with minimum essential medium non-essential amino acids;
(2) the vitamins comprise one or more of vitamin E, vitamin B12, and vitamin C;
(3) the inorganic salts comprise one or more of sodium chloride and sodium selenite.

12. The induction medium according to claim 11, wherein the induction medium satisfies one or more of the following conditions:
(1) the growth factors comprise vitamins, 2.0 ng/mL to 15 ng/mL progesterone, 2 µg/mL to 50 µg/mL 1,4-butanediamine, and 5 mg/L to 40 mg/L insulin, and the vitamins comprise 0.1 µg/mL to 15 µg/mL vitamin E, 0.5 µM to 2.4 µM vitamin B12, and 32 mg/L to 128 mg/L vitamin C;
(2) the inorganic salts comprise 0.5 g/L sodium chloride and 13.6 µg/L sodium selenite;
(3) the additives comprise 10 µM to 200 µM piperidinol oxide, 5 µM to 150 µM luteolin, 5 µg/mL to 25 µg/mL D(+)-galactose, and 50 ng/mL to 200 ng/mL recombinant human transferrin.

13. A method for inducing the differentiation of pluripotent stem cells into neural lineage cells, comprising the step of:
adherently culturing pluripotent stem cells with the induction medium according to any one of claims 1 to 12 to obtain neural lineage cells.

14. Use of the BMPR1B inhibitor in the induction medium according to any one of claims 1 to 12 in inducing the differentiation of pluripotent stem cells into neural lineage cells.
